# EUROPEAN PATENT APPLICATION

(11) **EP 3 449 815 A1**
(43) Date of publication of application: **06.03.2019**
(21) Application number: 18191074.6
(22) Date of filing: 28.08.2018
(51) Int. Cl.: A61B 5/00, A61B 18/20, A61B 18/00

(54) **MONITORING OF TISSUE COAGULATION BY OPTICAL REFLECTANCE SIGNALS**

(30) Priority: 28.08.2017 DE 102017119697
(71) Applicant: Fraunhofer-Gesellschaft zur Förderung der angewandten Forschung e.V., 80686 München (DE); University of Latvia, 1586 Riga (LV); LifePhotonic GmbH, 53129 Bonn (DE)
(72) Inventor: Wehner, Martin, 52134 Herzogenrath (DE); Spigulis, Janis, 1046 Riga (LV); Lihacovs, Aleksejs, 1048 Riga (LV); Trebst, Tilmann, 53123 Bonn (DE)
(74) Representative: Gagel, Roland

(57) **Abstract**

The invention relates to a method and an apparatus for assessing coagulation of tissue (1) during heat treatment, comprising at least one radiation source (2) emitting light; at least one illumination optical means (6) coupled to the at least one radiation source (2) for directing the emitted light to a predetermined region of the tissue (1); at least one detection optical means (8) for collecting light backscattered by a surface or surface layer of the predetermined region of the tissue (1); and a detecting and analyzing unit (3, 4) coupled to the at least one detection optical means (8) for detecting and analyzing the light backscattered by the tissue (1); wherein the detecting and analyzing unit (3, 4) is adapted to detect intensities of backscattered light of at least two different wavelengths or wavelength regions in a spectral range between 400 nm and 800 nm and to derive an assessment value on the basis of the detected intensities, which assessment value is an indicator for the coagulation status of the tissue (1) . The invention further relates to an apparatus for coagulation of tissue by heat treatment using such an assessing apparatus.

## Description

The present invention relates to a method and an apparatus for assessing coagulation of tissue during heat treatment and to an apparatus for coagulation of tissue by heat treatment using such an assessing apparatus.

Precise control of tissue modifications during heat induced treatments is often necessary to ensure a sufficient treatment outcome on one hand, while often an overtreatment may result in detrimental side effects. A typical example is laser induced tissue welding or tissue soldering where the coagulation of proteins, e.g. albumins, leads to a fixation of two tissues or a fixation of a bandage, stent or similar to a tissue. For the process, a sufficient temperature needs to be reached for a specific amount of time, while an overheating of the surrounding tissue shall be avoided in order to avoid too large coagulated lesions, necrosis or even carbonization. Typically, a temperature beyond 40°C and below 100°C needs to be reached. A more precise range e.g. 65 to 85°C might be preferable for specific proteins.

Laser tissue soldering (LTS) and laser tissue welding (LTW) are two similar procedures for photo-thermal tissue bonding. In laser tissue welding, a thermally induced cross linking of tissue proteins can be obtained after approximation of the edges of an incision and laser irradiation. In laser tissue soldering, a protein solution is added which serves as glue or "solder" between a wound surface and a dressing or patch. The protein solution is thermally cross-linked and bonds the dressing to the tissue. Due to reduced thermal damage to tissue, LTS is often preferred over LTW. Nevertheless, a thin tissue layer in the wound area is affected by the heat treatment. Laser tissue interaction is being investigated since many years.

Typically, for LTS and LTW tissue is heated up to a sufficiently high temperature for a few seconds duration. This results in denaturation and cross-linking of proteins, while the water content is reduced and tissue shrinks. In contrast to tumor treatment, where often a large coagulation depth of millimeters to centimeters is required, the thickness of the heat affected zone and the extension of the coagulation zone must be limited for LTS and LTW. The thickness of the heat affected tissue zone should be low, e.g. below 1 mm, not to impair wound healing. A major challenge in LTS and LTW is to minimize the depth of the heat affected zone and to limit the heat input into tissue while ensuring a sufficiently high bonding strength which requires coagulation.

In this context, reference is made to DE 10 2012 002 818. That document discloses a device which can be used for LTS as well as for LTW.

Since tissues exhibit a great variety of composition and morphology, the optical properties could change from sample to sample which influences absorption of laser light and the heating rate. Therefore, usually temperature sensors are used to monitor the temperature and the laser power can be controlled to regulate the maximum temperature. Even though temperature sensors and ear thermometer are available at low costs, the requirements for a surgical procedure are more demanding. A sufficiently fast response of the sensor is required. Own experiments show that for application of a laser power of up to 40 W on a spot of 5 mm in diameter, the rise time of the sensor should not exceed 10 ms. Otherwise laser shut down will be too slow before carbonization starts. Furthermore, when working in confined space, e. g. during a minimally invasive intervention, the size of the temperature sensor must be sufficiently small to be integrated into an instrument.

EP 2 015 672 B1 discloses a method and an optical detection system for evaluation of tissue modifications. The system comprises an ablation catheter, one or more radiation sources operable at predetermined wavelengths, a plurality of optical fibers disposed within the ablation catheter for directing radiation from the radiation sources to one or more targeted tissue components and for receiving and directing an induced backscattered radiation, a device for recording one or more spectra of the backscattered radiation and means for analyzing one or more spectral changes of the spectra. The method detects tissue destructions as they are typically achieved during cardiac tissue ablation. The method relies on backscattered light in the NIR wavelength range (600- 1500nm) for recognizing charring, the formation of coagulum, evaporating water or even steam pops. All these processes require intervention by significant amounts of heat or significant cooling in case of cryosurgery and result in significant modifications of the tissue.

A similar method and apparatus for monitoring the ablation of cardiac tissue with an ablation catheter is described in US 2017/0202619 A1. The document mainly refers to the NIR wavelength range for the monitoring, but also proposes to use a ratio between a reflectance signal at a wavelength of 800 nm and a reflectance signal at a wavelength of interest ranging between 400 nm and 2500 nm. For the monitoring of the ablation process the light backscattered from deeper regions of the tissue, at least 5 mm depth, must be collected. This is achieved by a substantial spatial offset between the fibers for illumination and collecting the light.

Coagulation processes as occurring during LTS and LTW require a much more sensitive method allowing the detection of more subtle modifications to the upper layers of tissue.

It is an object of the present invention to provide such a method and apparatus for assessing coagulation of tissue during heat treatment.

This object is solved by a method as defined in claim 1 and by an apparatus as defined in claim 9 and an apparatus as defined in claim 15. Advantageous configurations of the method as well as of the device are defined in the dependent claims or can be drawn from the following description as well as from the exemplary embodiments.

It has been detected that utilizing the visible wavelength range for the monitoring provides for a sensitive measurement of subtle changes. Coagulation of tissue leads to an increasing in scattering of light. This scattering is linked to the denaturation of proteins and visible to the naked eye.

Characteristic spectral changes occur when the temperature of tissue samples increase above 70 °C which is a typical setpoint value for temperature control of coagulation. It is concluded that simple spectroscopy in the visible range can provide valuable information during LTS and LTW and replace or supplement the delicate measurement of temperature. A major advantage is that optical measurements can be performed using standard optical fibers and can be easily integrated into a surgical tool. Furthermore, utilizing the visible wavelength range for monitoring is advantageous, as the method is compatible with all typical wavelengths used in laser surgery, especially 808nm, 810nm, 940nm, 960nm - 980nm, 1064nm, 1320nm, 1470nm.

In the proposed method a predetermined region of the tissue is illuminated with light. The light backscattered from a surface or (thin) surface layer of the predetermined region of the tissue is collected. Intensities of the backscattered light of at least two different wavelengths or wavelength regions in a spectral range between 400 nm and 800 nm, preferably between 500 nm and 700 nm are detected and an assessment value is derived on the basis of the detected intensities, which assessment value is an indicator for the coagulation status of the tissue.

In a preferred embodiment the first of the two different wavelengths or wavelength regions is selected from the wavelength range between 525 nm and 585 nm and the second of the two different wavelengths or wavelength regions is selected from the wavelength range between 600 nm and 650 nm. It has been recognized that such a combination of wavelengths/wavelength regions achieves a specifically high sensitivity which is advantageous for monitoring of tissue coagulation.

Depending on the site of application different optical setups may be used. On easily accessible sites as the skin free-space optical setups may be used. When targeting sites inside the body either through natural body cavities such as the mouth, endoscopically, or through surgical openings, optical setups with one or more optical fibers are used. Several variations are feasible. One option, the most compact, is to use a single fiber for guiding the light towards the site and for collecting and guiding the backscattered light back towards the spectral analysis. It requires beam combining and separation optics on the instrument side of the fiber. In another option, several fibers can be used to separate different functions, e.g. one fiber for guiding light towards the site and one for collecting the backscattered light. Preferably the optical axes of the two optical fibers at their fiber ends facing the predetermined region of the tissue are arranged slightly tilted by an angle < 20°, preferably between 5° and 15°, normal to the surface of the predetermined region of the tissue in order to mainly collect light backscattered from a thin surface layer of the tissue. As described below the light for monitoring can be a light source with a rather broad spectral range, e.g. a white-light source, but also a combination of light sources with a rather narrow spectral range, e.g. a few 10 nm spectral width or even less. The light of these spectrally narrow wavelengths could be guided through a common fiber as well as through a dedicated fiber for each light source. The combined embodiment of all fibers is often called a fiber-optic probe.

Preferably the illumination of the predetermined region and the collection of the backscattered light are performed such that mainly light backscattered from a surface layer of a thickness of < 1 mm is collected, since the optical effects for monitoring the coagulation process occur at the surface or in such a thin surface layer.

In one embodiment, rather broadly emitting light sources such as lamps, halogen lamps, white-light LEDs or similar may be used to illuminate the tissue with a subsequent spectral analysis of the reflected light. Such analysis may be accomplished with spectrometers, but also with filters (high/low/bandpass) and light detectors such as photodiodes, photomultipliers, CCD or the like. This embodiment leaves the largest freedom in data analysis.

Alternatively, in another preferred embodiment, the spectral selection of the light takes place prior to the illumination of the tissue which allows for simpler detection schemes without an additional filtering of the light. The spectral selection of the illuminating light may be accomplished by combining white light sources with filters or by applying light sources with a narrow spectral width such as LEDs or lasers.

Concerning the analysis of the data, there are several options. Above method provides intensity values for the backscattered light for at least one spectral range. This might be e.g. the reading of a photodiode while illuminating with a single, one color LED, or the area under a dedicated part of a spectrum acquired with a spectrometer. When using dedicated light sources with narrow spectral ranges and only a single photodetector, a switching pattern between light sources needs to be applied in order to distinguish the backscattered light signals.

Taking ratios of 2 such values is beneficial as the ratio cancels out numerous wavelength independent influences on the absolute height of the signal, e.g. the distance of the probe to the targeted tissue, the angle between probe and tissue, etc. The ratio thus becomes independent of these influences. Furthermore, each intensity value might even be a ratio by itself taking the intensity signal of the backscattered and normalizing it by an intensity signal taken close to the light source in order to cancel out intensity fluctuations of the light source.

It is also possible to take the difference of two intensity signals. Again it might be advantageous of normalizing this difference with other intensity signals to cancel out fluctuations of the light source(s) or to cancel out influences along the light path as described above.

In a preferred embodiment a maximum of said difference or ratio of intensity signals during the heat treatment is detected. This maximum is an indication that the maximum temperature for the coagulation tolerable without destruction of surrounding tissue has been reached and can be used for the control of the heating apparatus to stop the heat treatment.

The proposed apparatus for assessing coagulation of tissue during heat treatment comprises a detecting and analyzing unit adapted to detect intensities of backscattered light of at least two different wavelengths or wavelength regions in a spectral range between 400 nm and 800 nm, preferably between 500 nm and 700 nm, and to derive an assessment value on the basis of the detected intensities according to the proposed method. In preferred embodiments this apparatus is designed to perform the preferred embodiments of the method as described above or in claims 2 to 8.

These and other features and advantages will be better understood by reading the following detailed description, taken together with the drawings wherein:

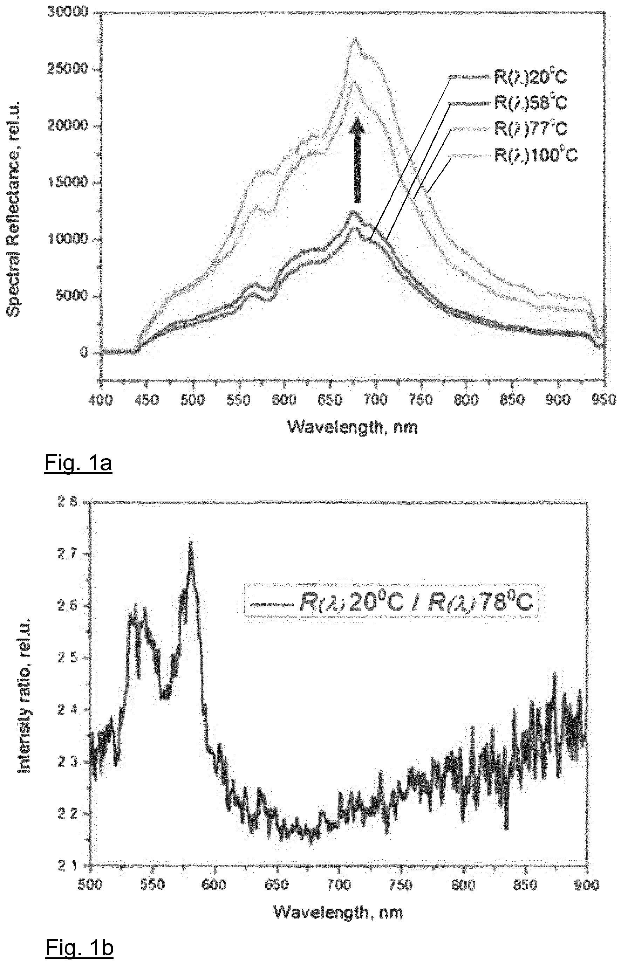

- Figure 1 a): shows increase of spectral reflectance with increasing temperature,
- Figure 1 b): shows spectrally resolved ratio of reflectance signal at 78 °C to signal at 20°C; porcine muscle tissue, 980 nm, 30 W, spot size 10 mm, dwell time 20s.
- Figure 2.a): shows correlation of difference in reflectance signal from 620 nm - 580 nm wavelength with temperature at three different spots,

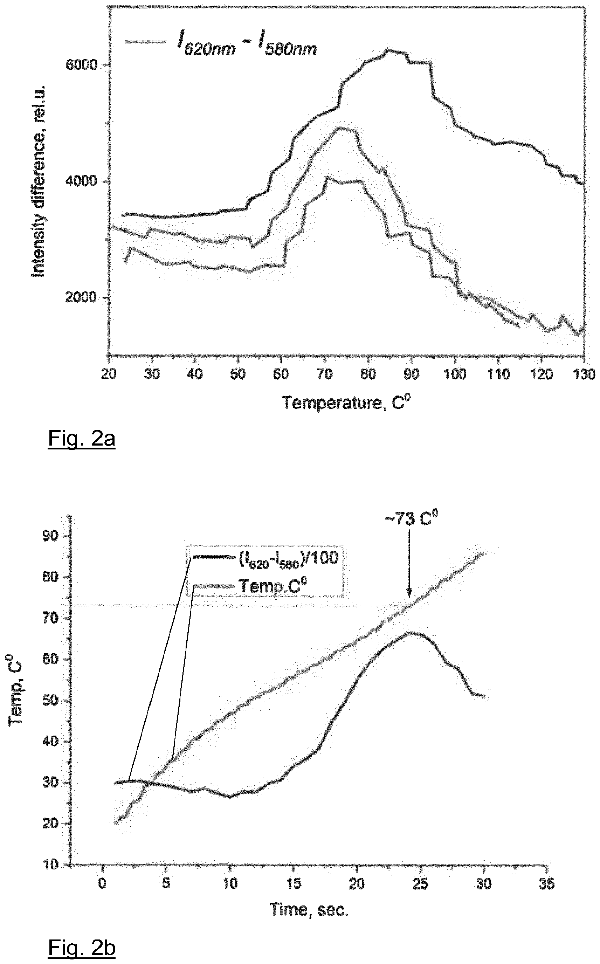

- Figure 2 b): shows time course of difference signal and temperature increase for irradiation parameters of fig.1.

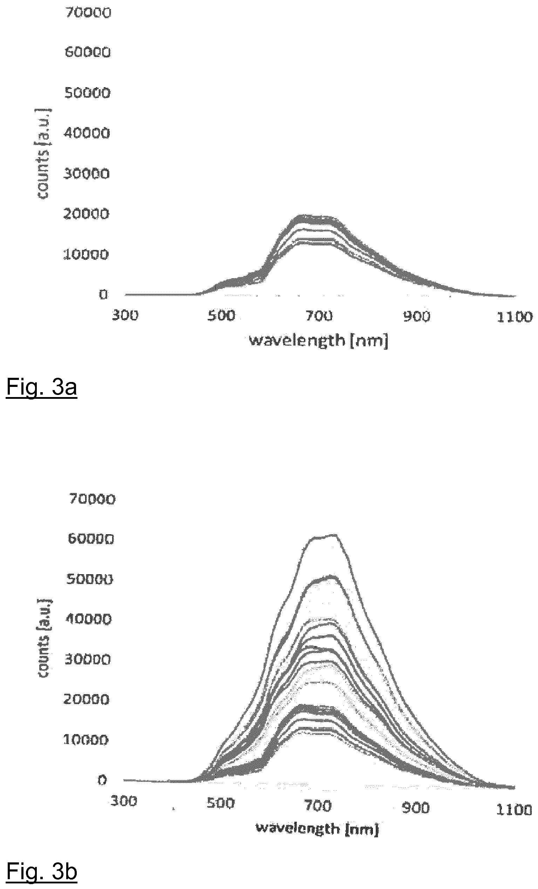

- Figure 3 a): shows reflectance spectra from 12 samples of porcine hog jowls in native state.
- Figure 3 b): shows reflectance spectra from 12 samples of porcine hog jowls after coagulation at 75 °C setpoint temperature for 5 s.

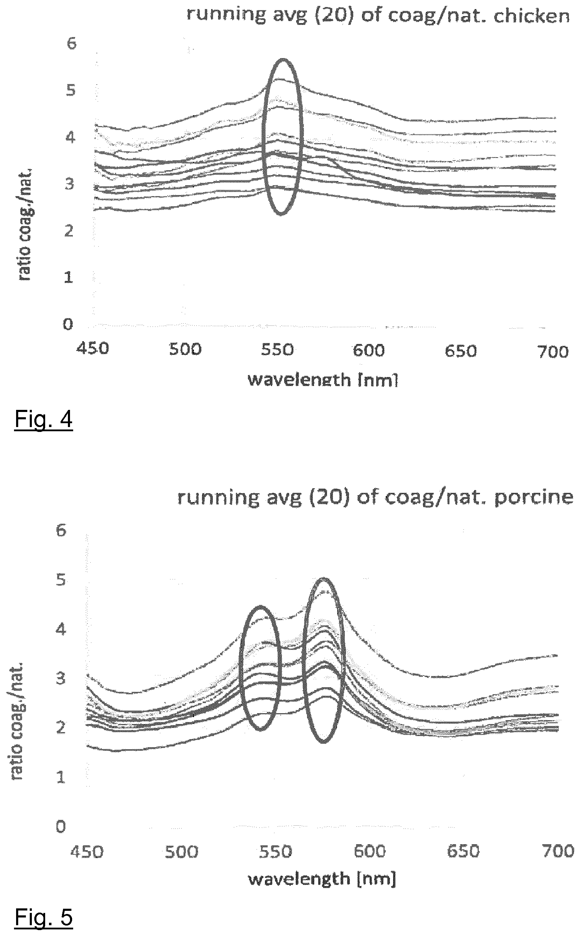

- Figure 4: shows ratio of spectral reflectance Rcoa./ Rnat. for coagulated chicken breast, a slight peak appears at 550 nm wavelength.
- Figure 5: shows ratio of spectral reflectance Rcoa./ Rnat. for coagulated porcine hog jowls, two peaks appear at 530 nm and 580 nm wavelength.

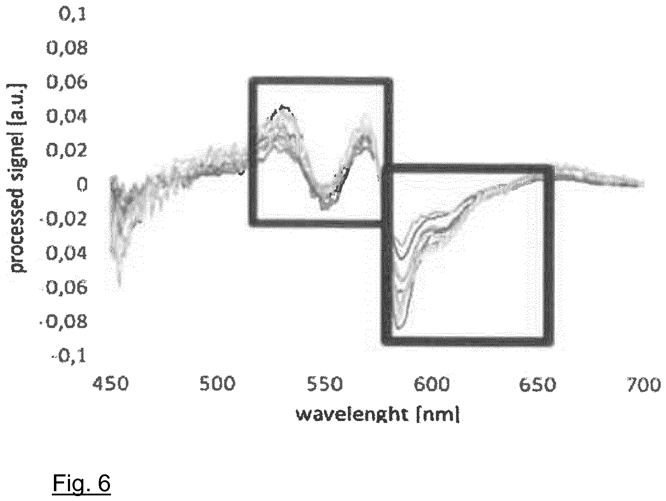

- Figure 6: shows first derivative of the spectrum of fig. 5, the peaks at 530 nm and 570 nm and the dip at 585 nm are typical for coagulation of porcine hog jowl tissue.

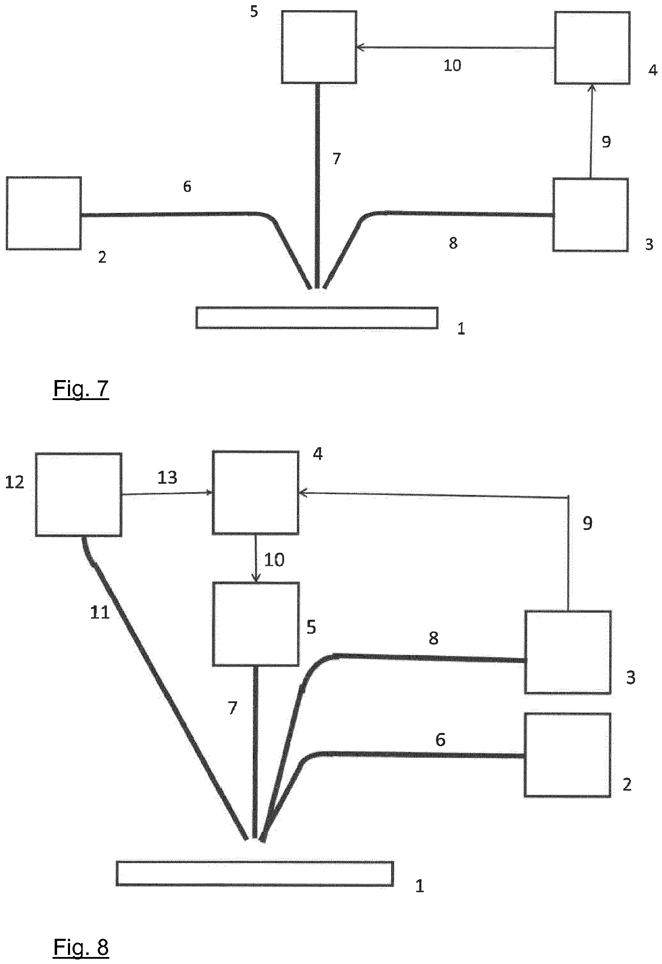

- Figure 7: shows a schematic view of an apparatus for coagulation of tissue by laser treatment according to an embodiment of the present invention..
- Figure 8: shows a schematic view of an apparatus for coagulation of tissue by laser treatment according to another embodiment of the present invention.

### Materials and Methods

The applicant has carried out several experiments using the following setup for irradiation at fixed laser power:
Tissue samples are irradiated by a diode laser operating at 980 nm at maximum power of 30 W. The laser radiation is coupled into a 400 µm core fiber of a bifurcated fiber bundle, and the reflectance signal is captured by six 200 µm core fibers and fed to a spectrometer. The spectrometer is sensitive in a wavelength range of 400 - 950 nm, the spectral resolution is about 2 nm. Additionally a short pass filter with edge at 950 nm is mounted before the entrance of the spectrometer to avoid saturation of the detector.

The end face of the bare fiber bundle is positioned in 15 - 25 mm distance to the tissue sample which results in a spot size of up to 10 mm in diameter. Then the laser is switched on for 20 s to heat up the sample, and spectra are recorded during irradiation. Illumination is achieved by a halogen light bulb which is coupled to a 200 µm single core fiber, the distance and the angle of the fiber end are adjusted to fit the laser spot size. Additionally, a thermal imager in the long wavelength range with a pixel resolution of 640 x 480 is used to record the temperature of porcine muscle tissue, the samples are obtained from a local butcher.

Fig. 7 shows a schematic view of an example of such an apparatus for coagulation of tissue by laser treatment. The tissue sample 1 is irradiated by laser source 5 with fiber coupling 7 and power control for local heating of the tissue. The heated area is at the same time illuminated with a light source 2, e.g. a white light or multi wavelength LED source, via illumination fiber or fiber bundle 6. The reflectance signal is captured by fiber or fiber bundle 8 and fed to a spectral detector 3, e.g. a spectrometer or bandpass filtered photo diodes. The data on the spectral composition of the collected back-scattered light 9 are transferred to a microprocessor or computer 4 for evaluation according to the proposed method. Based on this evaluation the microprocessor or computer 4 then generates a signal 10 for power control of the laser source 5.

The following setup has been used for temperature controlled irradiation:
The experimental set up consists of a fiber-coupled double-bar laser diode module equipped with 980 nm and 1540 nm diode bars, a controller with power supplies. The 980 nm laser bar provides a maximal output power of 45 W and the 1540 nm bar of 12 W. The radiation of both bars is fed into the same optical fiber with a core diameter of 600 µm, and an optical lens is used to project a 8 to 10 - fold magnified image of the fiber end face onto the tissue sample.

The surface temperature is monitored by an infrared sensor with a response time of less than about 2 ms. The temperature signal is delivered to a laser diode controller which comprises two independent power supplies with integrated digital PID- controller for both laser diode bars. The temperature signal is compared to the setpoint value and the laser power adjusted accordingly. The power ratio of the two laser sources can either be fixed at a predefined value or altered during the time course. Here we prefer a procedure where first the 1540 nm laser source is activated for 3 s and then shut down followed by 2 s irradiation at 980 nm wavelength. The coagulation procedure is performed at a setpoint temperature of 75 °C to prevent excess heating of tissue. Because of the short response time of the sensor and the fast control circuit the laser power will be safely limited when the programmed temperature threshold will be exceeded.

Spectral data are recorded by a fiber coupled spectrometer in the range of 320 - 1100 nm with a spectral resolution of about 2.5 nm. All optical axis are arranged slightly tilted by 10° normal to the surface like edges of a pyramid and coincide in one focal point. A reference spectrum ("native") is acquired before the laser is activated, after irradiation the spectra of coagulated spots ("coagulated") are recorded. Investigations are performed on freshly slaughtered hog jowls which were stored at - 20 °C for 3 days and exposed to room temperature 12h before irradiation tests. Since hog jowl are well supplied with blood and appear as a relatively dark red tissue, chicken breast are chosen for comparison as light tissue.

Fig. 8 shows a schematic view of an example of such an apparatus using power control by temperature sensing and evaluation of the spectral signal. In addition to the components of the apparatus of fig. 7 an optical fiber or fiber bundle 11 is arranged for transmission of temperature radiation of the sample to a pyrometer 12 for temperature detection. The temperature signal 13 of the pyrometer 12 is fed to the microprocessor or computer 4 for power control of the laser source 5 based on the temperature sensing and on the evaluation of the spectral signal.

### Experimental Results

### Measurements at fixed laser power

The experiments reveal that during irradiation temperature increases and reflectance increases, too. The reflectance signal rises by a factor of about 2.5 and slopes look similar, but the changes are not equal in each spectral band (see fig.1). A sharp rise can be observed in 520 - 600 nm spectral range where the rise at 630 nm is clearly lower. It is obvious that between 58 °C and 77 °C a strong change occurs. The correlation of spectral features with temperature increase can be visualized when the difference (amount) between the signals at (620 ± 2) nm and (580 ± 2) nm is plotted versus temperature. Due to variation of tissue morphology and probe orientation at different spots the absolute values differ, but a common feature is the steep signal increase between 70 - 80 °C temperature (see fig.2). The time course of difference signal and temperature signal suggests that morphological changes occur above 70 °C temperature but diminishes when exceeding 80 °C. The dynamic change of the signals is between 150-180 % which is easily to detect.

### Measurements under temperature control

The spectral changes in the signature range between 450 - 750 nm wavelength were investigated on a set of 12 samples to evaluate which signal fluctuations can be expected. When comparing the signal strength of native with coagulated samples (see fig. 3) it is obvious that the slope is similar, but variations in signal height are lower for native than for coagulated samples. Looking at the plot of signal ratio over wavelength for chicken, values between 2.5 to 5.5 occur, and the results obtained for hog jowls reveal a lower factor of 1.5 to 4.5 - fold (see fig. 4). In case of porcine tissue the slope exhibits more features and a clear double peak at 530 nm and 580 nm wavelength appears (see fig. 5). It is clear from these observations that not only signal height but signal ratio can be very different as well.

The detection of coagulation can be performed on an absolute level regarding the ratio signals from native and coagulated tissue. But changes in spectral characteristics become more pronounced when the first derivative of the ratio plots is considered (see fig. 6). A typical structure of two peaks at 530 nm and 570 nm, and the dip at 585 nm appears.

### Discussion

Acquisition of reflectance data in the region 500 - 700 nm reveals information on the coagulation state. A relatively simple data analysis can be performed by determining the difference of two spectral bands which are relevant as depicted in fig. 2. The difference signal increases through coagulation by 50% - 80% which can be easily detected. By analysis of the signal slope endpoint detection may become feasible, when the curve drops off the useful temperature range is exceeded and the laser should be shut down. Signal processing can be performed by using white light source and photodiodes with bandpass filters or simply by LEDs or small diode lasers in combination with a single photo detector. The control functions can be realized by analog circuits which facilitates certification and adherence to safety aspects for a medical device.

A second approach described in figs. 4 and 5 when the ratio of spectral data of coagulated reflectance data to native spectral data is considered. Depending on the type of tissue, e.g. light tissue with low hemoglobin concentration or well perfused tissues, a 2 to 5 - fold signal increase can be expected in a signature range from 500 nm to 600 nm wavelength. This can be detected by simple filtering and analog circuits making online control of laser power feasible. Further, comparing the signal in two spectral bands, e. g. near 620 nm and 580 nm wavelength, the relation of the signals is an indicator for coagulation of tissue. The spectral signal allows for 20% to 50% discrimination depending on tissue type. A very important advantage here is that the signal ratio is independent from of the absolute level of the received light. Arbitrary signals induced by involuntary movements or shrinkage of tissue cannot deteriorate analysis of signals.

Spectral features are stronger highlighted when the first derivative of coagulated / native signal ratio is calculated as depicted in fig. 6. The spectral region between 500 nm and 650 nm shows a pronounced structure with a signature in 520 - 630 nm spectral range. The variation range of signals obtained from 12 different samples are less than 50% of the mean signal which should allow for a sensitive and reliable detection of coagulation. However, the methodology requires acquisition and handling of a data set of at least 200 data points, and the fast processing of the spectral data. In principle, this is feasible by using cheap microcontrollers, but algorithms and hardware for numerical data processing (acquisition, averaging, calculation of ratio, smoothing) must be evaluated under safety aspects for maximum reliability. The investigations show that dark tissue supplied with blood gives a more pronounced reflectance signal during coagulation than light tissue. This is possibly caused by the decomposition of hemoglobin. The experiments on porcine hog jowls exhibit peaks at 530 nm and 570 nm, and a dip at 585 nm wavelength. This may be attributed to spectral characteristics of hemoglobin which show peaks at 540 / 560 / 580 nm depending on oxygen saturation index.

### List of reference signs

- 1: tissue sample
- 2: light source, white light or multi wavelength LED source
- 3: Spectral detector, e.g. spectrometer or bandpass filtered photo diodes
- 4: microprocessor or computer
- 5: laser source with fiber coupling and power control
- 6: illumination fiber / bundle
- 7: laser fiber
- 8: fiber / bundle for collecting back-scattered light
- 9: data on spectral composition of spectral back-scattered light
- 10: signal for power control of laser source
- 11: optical fiber / bundle for transmission of temperature radiation
- 12: pyrometer for temperature detection
- 13: temperature signal for power control of laser source

## Claims

1. A method of assessing coagulation of tissue during heat treatment, in particular during laser induced tissue welding or tissue soldering, comprising the steps of
illuminating a predetermined region of the tissue (1) with light; collecting light backscattered from a surface or surface layer of the predetermined region of the tissue (1); detecting intensities of backscattered light of at least two different wavelengths or wavelength regions in a spectral range between 400 nm and 800 nm, preferably between 500 nm and 700 nm; and
deriving an assessment value on the basis of the detected intensities, which assessment value is an indicator for the coagulation status of the tissue (1).

2. The method of claim 1, wherein
deriving an assessment value comprises determining the difference between the detected intensities at two different wavelengths or the integrated intensities of two different wavelength regions.

3. The method of claim 1, wherein
deriving an assessment value comprises determining the ratio of the detected intensities at two different wavelengths or the integrated intensities of two different wavelength regions.

4. The method of claim 2 or 3,wherein
a maximum of said difference or ratio during the heat treatment is detected.

5. The method of any of claims 1 to 4, wherein
a first of the two different wavelengths or wavelength regions is from the wavelength range between 525 nm and 585 nm and a second of the two different wavelengths or wavelength regions is from the wavelength range between 600 nm and 650 nm.

6. The method of any of claims 1 to 4, wherein
the two different wavelengths are about 580 nm and about 620 nm.

7. The method of any of claims 1 to 6, wherein
the illumination of the predetermined region and the collection of the backscattered light are performed such that mainly light backscattered from a surface layer of a thickness of < 1 mm is collected.

8. The method of any of claims 1 to 7, wherein
the illumination is performed with at least one first optical fiber (6) and the collection of the backscattered light is performed with at least one second optical fiber (8), optical axes of said first and second optical fibers (6, 8) at fiber ends facing the predetermined region of the tissue (1) being arranged slightly tilted by an angle < 20°, preferably between 5° and 15°, normal to the surface of the predetermined region of the tissue (1).

9. An apparatus for assessing coagulation of tissue during heat treatment, in particular during laser induced tissue welding or tissue soldering, comprising
at least one radiation source (2) emitting light;
at least one illumination optical means (6) coupled to the at least one radiation source (2) for directing the emitted light to a predetermined region of the tissue (1); at least one detection optical means (8) for collecting light backscattered from a surface or surface layer of the predetermined region of the tissue (1); and
a detecting and analyzing unit (3, 4) coupled to the at least one detection optical means (8) for detecting and analyzing the light backscattered by the tissue (1);
wherein the detecting and analyzing unit (3, 4) is adapted to detect intensities of backscattered light of at least two different wavelengths or wavelength regions in a spectral range between 400 nm and 800 nm, preferably between 500 nm and 700 nm, and to derive an assessment value on the basis of the detected intensities, which assessment value is an indicator for the coagulation status of the tissue (1).

10. The apparatus of claim 9, wherein
the apparatus comprises two radiation sources (2), each emitting light of a narrow spectral width.

11. The apparatus of claim 9 or claim 10, wherein
the detecting and analyzing unit (3, 4) comprises a photodiode for detecting light intensities and a microprocessor.

12. The apparatus of claim 9 or claim 10, wherein
the detecting and analyzing unit (3, 4) consists of analogue electronics.

13. The apparatus of any of claims 9 to 12, wherein the illumination and detection optical means (6, 8) comprise light guiding means, preferably optical fibers.

14. The apparatus of any of claims 9 to 13, wherein
the detecting and analyzing unit (3, 4) is adapted to detect intensities of backscattered light of a first of the two different wavelengths or wavelength regions in the wavelength range between 525 nm and 585 nm and of a second of the two different wavelengths or wavelength regions in the wavelength range between 600 nm and 650 nm,

15. An apparatus for coagulation of tissue by heat treatment, in particular by laser induced tissue welding or tissue soldering, comprising a heating unit, preferably a laser unit (5), for applying heat to a predetermined region of the tissue (1);
an apparatus for assessing coagulation of tissue during heat treatment as defined in any of claims 9 to 14; and
a control unit for controlling the heating unit in response to the assessment value computed by the assessing device.
